# EUROPEAN PATENT APPLICATION

(11) **EP 4 796 041 A1**
(43) Date of publication of application: **26.08.2026**
(21) Application number: 24879887.8
(22) Date of filing: 12.07.2024
(51) Int. Cl.: A61B 6/00, A61B 6/50, A61B 6/03, G06T 7/10, G06T 7/30

(54) **METHOD AND SYSTEM FOR QUANTITATIVELY ANALYZING BRAIN IMAGE BASED ON CT IMAGE**

(30) Priority: 20.10.2023 KR 20230141203
(71) Applicant: Newcure M Inc., Seoul 03722 (KR)
(72) Inventor: CHOO, Kyobin, Seoul 03722 (KR)
(74) Representative: Studio Torta S.p.A.
(86) International application number: PCT/KR2024/010017
(87) International publication number: WO 2025/084546

(57) **Abstract**

A method for quantitative analysis of brain images based on computed tomography (CT) images performed by at least one processor is provided. The method for quantitative analysis of brain images includes receiving a CT image and a positron emission tomography (PET) image of a same patient from an external device, registering the CT image and the PET image, extracting at least one region of interest included in the CT image using a CT parcellation model, and calculating an analysis result for the at least one region of interest.

## Description

### Technical Field

The present disclosure relates to a method and a system for quantitative analysis of brain images based on computed tomography (CT) images, and specifically, to a method and a system for extracting an anatomical region of interest from a brain CT image using a CT parcellation model and calculating a brain quantitative analysis result of the corresponding region of interest.

### Background

To accurately determine cancer, brain diseases, and the like, it is desirable for medical institutions to capture Positron Emission Tomography (PET) and Magnetic Resonance Imaging (MRI) images from a body of a subject (e.g., a patient). Here, a CT image may generate a cross-sectional image of the body of the patient captured from various angles using an X-ray rotor, and a PET image may generate an image by investigating metabolic activity of the body using radioactive isotopes. Furthermore, an MRI image may generate a tissue image of the body of the patient using a strong magnetic field and radio waves.

In performing quantitative analysis of medical images, a CT image and an MRI image may provide complementary information to each other. For example, the CT image may clearly show a bone or a skeletal structure of the patient, whereas the MRI image may more accurately show soft tissues and a nervous system. Therefore, it may be possible to make a more accurate diagnosis and take a more effective action by using the CT image and the MRI image together. However, because of safety reasons, there may be a patient who cannot undergo MRI, such as a patient using a metal implant or a pacemaker. Also, in a case where the CT image and the MRI image are used together, there is a problem that costs and time for the patient are consumed significantly because not only the CT image but also the MRI image must all be captured.

Furthermore, because medical institutions at home and abroad mainly use a PET/CT device in which a PET image and a CT image are generated together rather than a PET/MRI device in which a PET image and an MRI image are generated together when performing a PET examination. Accordingly, when the PET image is captured, the CT image is often generated together.

### DETAILED DESCRIPTION

### Technical Problem

The present disclosure provides a method and a system for quantitative analysis of brain images based on CT images for solving the problems as described above.

### Technical Solution

The present disclosure may be implemented in various ways including a method, a device (system), or a computer program stored in a readable storage medium.

According to an embodiment of the present disclosure, in a method for quantitative analysis of brain images based on computed tomography (CT) images performed by at least one processor, the method may include receiving a CT image and a positron emission tomography (PET) image of a same patient from an external device, registering the CT image and the PET image, extracting at least one region of interest included in the CT image using a CT parcellation model, and calculating an analysis result for the at least one region of interest.

According to an embodiment of the present disclosure, the calculating of the analysis result may include calculating a standardized uptake value ratio (SUVR) of the at least one region of interest.

According to an embodiment of the present disclosure, the method may further include training the CT parcellation model using a predetermined training data set, and the predetermined training data set may be generated based on a CT image and an MRI image of the same patient.

According to an embodiment of the present disclosure, the training of the CT parcellation model using the predetermined training data set may include receiving the CT image and the MRI image of the same patient, generating labeling information of a plurality of regions included in the MRI image by performing a parcellation task on the MRI image, constructing a training data set with the labeling information and the CT image as a pair, and training the CT parcellation model using the training data set.

According to an embodiment of the present disclosure, the constructing of the training data set may include spatially registering the MRI image and the CT image.

According to an embodiment of the present disclosure, the training of the CT parcellation model may include applying a weight to each of a plurality of regions included in the MRI image, and the weight may be determined to be inversely proportional to an area of each of the plurality of regions included in the MRI image.

According to an embodiment of the present disclosure, the weight may be determined to be greater than or equal to a predetermined threshold.

According to an embodiment of the present disclosure, the CT parcellation model may include an axial view CT parcellation model, a coronal view CT parcellation model, and a sagittal view CT parcellation model, the axial view CT parcellation model may be trained based on an axial view CT image and labeling information of an axial view MRI image, the coronal view CT parcellation model may be trained based on a coronal view CT image and labeling information of a coronal view MRI image, and the sagittal view CT parcellation model may be trained based on a sagittal view CT image and labeling information of a sagittal view MRI image.

According to an embodiment of the present disclosure, the extracting of the at least one region of interest may include generating three-dimensional labeling information of the CT image by synthesizing respective labeling information generated by the axial view CT parcellation model, the coronal view CT parcellation model, and the sagittal view CT parcellation model.

A computer-readable non-transitory recording medium recording instructions for executing a method according to an embodiment of the present disclosure in a computer may be provided.

As a system according to an embodiment of the present disclosure, the system may include a communication module, a memory, and at least one processor connected to the memory and configured to execute at least one computer-readable program included in the memory, and the at least one program may include instructions for receiving a CT image and a PET image from an external device, registering the CT image and the PET image, extracting at least one region of interest included in the CT image using a CT parcellation model, and calculating an analysis result for the at least one region of interest.

### Advantageous Effects

According to various embodiments of the present disclosure, a CT parcellation model may generate labeling information at a level extracted from an MRI image using a CT image as an input value. That is, biological information having high resolution and contrast at a level obtainable from an MRI image may be acquired with only the CT image. Also, because an image required for better diagnosis may be obtained with only one CT scan, costs to be borne by a patient and time to be spent by the patient may be significantly reduced. Furthermore, because a CT image captured together while capturing a PET image through PET/CT equipment can be used, the CT image does not need to be captured separately from the PET image, so the patient costs and the time to be spent by the patient may be significantly reduced.

According to various embodiments of the present disclosure, the CT parcellation model may be trained by applying a weight determined to be greater than or equal to a predetermined threshold to each of a plurality of regions included in a brain MRI image. According to this configuration, the CT parcellation model may uniformly learn all regions regardless of a size of an area of a region extracted from the brain MRI image.

Effects of the present disclosure are not limited to the effects mentioned above, and other effects not mentioned will be clearly understood by those of ordinary skill in the art to which the present disclosure belongs (referred to as a "person of ordinary skill in the art") from the description of the claims.

### BRIEF DESCRIPTION OF THE DRAWINGS

Embodiments of the present disclosure will be described with reference to the accompanying drawings described below, wherein like reference numerals represent like elements, but are not limited thereto.
FIG. 1 illustrates an example of a method for quantitative analysis of brain images based on CT images according to an embodiment of the present disclosure.
FIG. 2 is a schematic diagram illustrating a configuration in which an information processing system is communicatively connected to a plurality of user terminals to quantitatively analyze brain images according to an embodiment of the present disclosure.
FIG. 3 is a block diagram illustrating internal configurations of a user terminal and an information processing system according to an embodiment of the present disclosure.
FIG. 4 illustrates an example of a method for training a CT parcellation model according to an embodiment of the present disclosure.
FIG. 5 illustrates an example of a method for training a CT parcellation model using two-dimensional brain images according to an embodiment of the present disclosure.
FIG. 6 illustrates an example of labeling information generated based on an MRI image and labeling information generated based on a CT image according to an embodiment of the present disclosure.
FIG. 7 is a flowchart illustrating an example of a method for quantitative analysis of brain images according to an embodiment of the present disclosure.

### DETAILED DESCRIPTION

Hereinafter, specific details for implementation of the present disclosure will be described in detail with reference to the accompanying drawings. However, in the following description, detailed descriptions of well-known functions or configurations will be omitted if the detailed descriptions may unnecessarily obscure the gist of the present disclosure.

In the accompanying drawings, the same or corresponding components are assigned the same reference numerals. Also, in the description of the following embodiments, redundant descriptions of the same or corresponding components may be omitted. However, even if the description of a component is omitted, such a component is not intended to be excluded from any embodiment.

Advantages and features of the disclosed embodiments and methods of achieving the advantages and features will become clear with reference to the embodiments described below in conjunction with the accompanying drawings. However, the present disclosure is not limited to the embodiments disclosed below but may be implemented in various different forms, and the present embodiments are only provided to make the present disclosure complete and to fully inform the scope of the invention to those of ordinary skill in the art.

Terms used in the present specification will be briefly described, and the disclosed embodiments will be described in detail. The terms used in the present specification have selected general terms that are currently widely used as much as possible while considering functions in the present disclosure, but the terms may vary according to intentions of technicians engaged in the relevant field, precedents, emergence of new technologies, or the like. Also, in a specific case, there are also terms arbitrarily selected by the applicant, and in this case, the meaning will be described in detail in the description part of the corresponding invention. Therefore, the terms used in the present disclosure should be defined based on the meaning of the terms and the contents throughout the present disclosure, rather than a simple name of the terms.

Singular expressions in the present specification include plural expressions unless the context clearly specifies otherwise. Also, plural expressions include singular expressions unless the context clearly specifies otherwise. Throughout the specification, when a part is said to include a certain component, this means that other components may be further included rather than excluding other components unless specifically stated otherwise.

Also, the term 'module' or 'part' used in the specification means a software or hardware component, and the 'module' or 'part' performs certain roles. However, the 'module' or 'part' is not limited to software or hardware. The 'module' or 'part' may be configured to be in an addressable storage medium or may be configured to reproduce one or more processors. Thus, as an example, the 'module' or 'part' may include at least one of components such as software components, object-oriented software components, class components, and task components, processes, functions, attributes, procedures, subroutines, segments of program code, drivers, firmware, microcode, circuits, data, databases, data structures, tables, arrays, or variables. Functions provided within the components and the 'modules' or 'parts' may be combined into a smaller number of components and 'modules' or 'parts' or further separated into additional components and 'modules' or 'parts'.

According to an embodiment of the present disclosure, a 'module' or 'part' may be implemented with a processor and a memory. A 'processor' should be interpreted broadly to include a general-purpose processor, a central processing unit (CPU), a microprocessor, a digital signal processor (DSP), a controller, a microcontroller, a state machine, and the like. In some environments, the 'processor' may also refer to an application-specific integrated circuit (ASIC), a programmable logic device (PLD), a field programmable gate array (FPGA), and the like. The 'processor' may also refer to a combination of processing devices, such as, for example, a combination of a DSP and a microprocessor, a combination of a plurality of microprocessors, a combination of one or more microprocessors combined with a DSP core, or any other such combination of configurations. Also, a 'memory' should be interpreted broadly to include any electronic component capable of storing electronic information. The 'memory' may also refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, and the like. If a processor can read information from and/or write information to a memory, the memory is said to be in electronic communication with the processor. A memory integrated into a processor is in electronic communication with the processor.

In the present disclosure, a 'system' may include at least one device among a server device and a cloud device, but is not limited thereto. For example, the system may be composed of one or more server devices. As another example, the system may be composed of one or more cloud devices. As yet another example, the system may be operated by configuring a server device and a cloud device together.

In the present disclosure, a 'display' may refer to any display device associated with a computing device, for example, any display device controlled by the computing device or capable of displaying any information/data provided from the computing device.

In the present disclosure, 'each of a plurality of A' or 'each of the plurality of A' may refer to each of all components included in the plurality of A, or may refer to each of some components included in the plurality of A.

In the present disclosure, a 'machine learning model' may include any model used to infer an answer for a given input. According to an embodiment, the machine learning model may include an artificial neural network model including an input layer, a plurality of hidden layers, and an output layer. Here, each layer may include a plurality of nodes. In the present disclosure, each of a plurality of machine learning models is described as a separate machine learning model, but is not limited thereto, and some or all of the plurality of machine learning models may be implemented as one machine learning model. Also, one machine learning model may include a plurality of machine learning models. In the present disclosure, the terms machine learning model and artificial neural network model may be used interchangeably to represent the same or similar models.

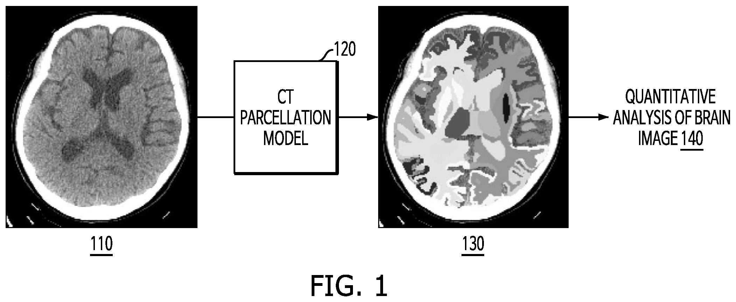

FIG. 1 illustrates an example of a method for quantitative analysis of brain images based on CT images according to an embodiment of the present disclosure. As illustrated, quantitative analysis 140 of a brain image may be performed based on a brain computed tomography (CT) image 110. Specifically, a CT parcellation model 120 may receive the brain CT image 110 as an input, extract a plurality of regions included in the brain CT image 110, and generate labeling information 130 for each region. Thereafter, a processor may perform the quantitative analysis 140 of the brain image based on the generated labeling information 130.

According to an embodiment, the processor may receive a brain image from an external device. Here, the external device may be a brain image capturing device. For example, the external device may be a CT device or a Positron Emission Tomography/Computed Tomography (PET/CT) device. Also, the brain image may include the brain CT image 110 and a positron emission tomography (PET) image.

According to an embodiment, the processor may pre-process the received brain image. For example, an intensity correction technique may be applied to the CT image to solve problems such as illumination non-uniformity, scattering, and noise occurring in the brain image. Also, various brain image correction techniques such as correcting patient movement in the PET image, correcting a scattering signal due to scattered particles, or filtering to reduce noise in the image may be used. Also, when sizes are different among brain images, the sizes among the brain images may be matched through scaling and/or re-sampling. Additionally or alternatively, various normalization techniques (e.g., Hounsfield Unit (HU)-based Min-Max scaling, Fuzzy C-means (FCM) tissue-based normalization, etc.) may be used to match a range of values obtained among the brain images. The above-described pre-processing techniques are only examples and are not limited thereto, and various pre-processing techniques may be used.

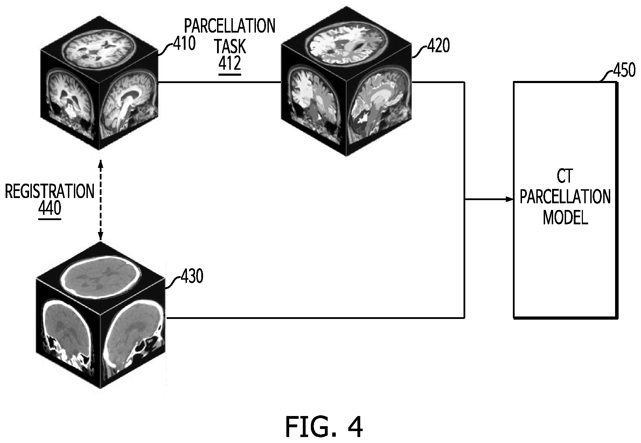

According to an embodiment, the processor may input the received brain CT image 110 into the CT parcellation model 120. The CT parcellation model 120 may be a model trained using, as a training data set, labeling information extracted by parcellating a CT image and an MRI image of a same patient. Here, the labeling information may include structural information of brain tissue. Accordingly, the CT parcellation model 120 may be trained to generate and output CT image-based labeling information 130 using the brain CT image 110 as an input value. Although FIG. 1 illustrates that the CT parcellation model 120 receives one brain CT image 110, the present disclosure is not limited thereto, and a plurality of brain CT images may be received. A method of the CT parcellation model 120 generating the labeling information 130 from the brain CT image 110 will be described later with reference to FIG. 4.

According to an embodiment, the processor may spatially register (co-register) the brain CT image 110 and the PET image into a same coordinate system. In FIG. 1, the brain CT image 110 is illustrated as a two-dimensional brain image, but the present disclosure is not limited thereto. For example, the brain CT image 110 may be a three-dimensional brain image. Alternatively, the brain CT image 110 may include an axial view brain CT image, a coronal view brain CT image, and a sagittal view brain CT image extracted from the three-dimensional brain CT image. Also, the PET image may be a three-dimensional brain image. Accordingly, the three-dimensional brain CT image and the three-dimensional PET image may be spatially registered in the same coordinate system in voxel units.

According to an embodiment, the brain CT image 110 and the PET image may be brain images of the same patient. Accordingly, the brain CT image 110 and the PET image may be registered by a rigid-body transformation. For example, the brain CT image 110 and the PET image may be registered through rotation and/or translation.

In an embodiment, the processor may spatially register (co-register) the brain CT image 110 and the PET image using a multi-modal coregistration algorithm. Specifically, to spatially register the brain CT image 110 and the PET image in the same coordinate system in voxel units, an optimal matrix (*T**) for mapping the CT image 110 to the PET image may be calculated. In this case, the optimal matrix (*T**) may be expressed as Equation 1 below. $T * = arg {max}_{T} {\sum }_{c} \left(p\left(X\left(c\right),Y\left(Tc\right)\right)log\frac{p \left(X\left(c\right),Y\left(Tc\right)\right)}{p \left(X\left(c\right)\right) p \left(Y\left(Tc\right)\right)}\right)$

Here, X may mean the CT image 110 and Y may mean the PET image. Also, c may mean each three-dimensional coordinate of the CT image 110. Also, p(X) may mean a probability density function for X, and p(X, Y) may mean a joint distribution for the probability density functions of X and Y. That is, when the CT image is rigidly transformed, the optimal matrix (*T**) that maximizes mutual information between the rigidly transformed CT image and the PET image may be calculated, thereby spatially registering the CT image 110 and the PET image in voxel units.

According to an embodiment, the processor may extract at least one anatomical region of interest based on the CT image-based labeling information 130 for the brain CT image 110 and the PET image. Thereafter, the processor may perform the quantitative analysis 140 of the brain image through PET image quantification for the anatomical region of interest. For example, the processor may calculate a standardized uptake value ratio (SUVR) of the region of interest. Accordingly, the processor may calculate an amyloid accumulation amount of the region of interest.

According to this configuration, the CT parcellation model 120 may generate labeling information 130 at a level extracted from a brain MRI image using the brain CT image 110 as an input value. That is, biological information having high resolution and contrast obtainable from a level of an MRI image may be acquired with only the CT image. Also, because an image required for better diagnosis may be obtained with only one CT scan, costs to be borne by the patient and time to be spent by the patient may be significantly reduced.

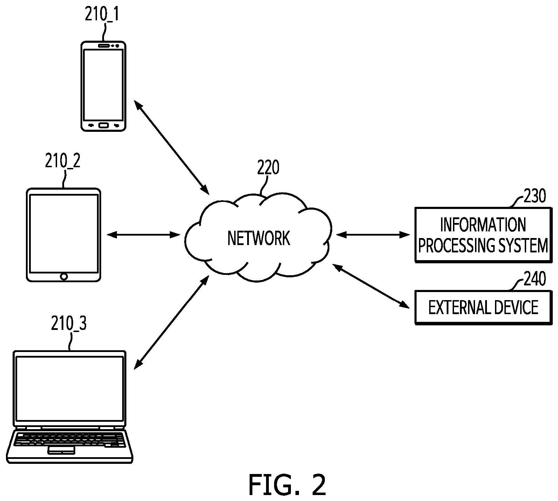

FIG. 2 is a schematic diagram illustrating a configuration in which an information processing system 230 is communicatively connected to a plurality of user terminals 210_1, 210_2, and 210_3 to quantitatively analyze brain images according to an embodiment of the present disclosure. As illustrated, the plurality of user terminals 210_1, 210_2, and 210_3 may be connected via a network 220 to the information processing system 230 capable of providing a brain image quantitative analysis service. Here, the plurality of user terminals 210_1, 210_2, and 210_3 may include a terminal of a user who is provided with the brain image quantitative analysis service.

In an embodiment, the information processing system 230 may include one or more server devices and/or databases capable of storing, providing, and executing computer-executable programs (e.g., downloadable applications) and data associated with providing the brain image quantitative analysis service, or one or more distributed computing devices and/or distributed databases based on cloud computing services.

The brain image quantitative analysis service provided by the information processing system 230 may be provided to the user through a brain image quantitative analysis service application, a web browser, or a web browser extension program installed on each of the plurality of user terminals 210_1, 210_2, and 210_3. For example, the information processing system 230 may provide information corresponding to a brain image quantitative analysis request received from the user terminal 210_1, 210_2, or 210_3 through the brain image quantitative analysis service application or perform a corresponding process.

The plurality of user terminals 210_1, 210_2, and 210_3 may communicate with the information processing system 230 via the network 220. The network 220 may be configured to enable communication between the plurality of user terminals 210_1, 210_2, and 210_3 and the information processing system 230. Depending on an installation environment, the network 220 may be configured as a wired network such as Ethernet, a wired home network (Power Line Communication), a telephone line communication device, and RS-serial communication, a wireless network such as a mobile communication network, WLAN (Wireless LAN), Wi-Fi, Bluetooth, and ZigBee, or a combination thereof. A communication method is not limited, and short-range wireless communication between the user terminals 210_1, 210_2, and 210_3 may also be included as well as a communication method utilizing a communication network (for example, a mobile communication network, wired Internet, wireless Internet, a broadcasting network, a satellite network, etc.) that the network 220 may include.

In FIG. 2, a mobile phone terminal 210_1, a tablet terminal 210_2, and a PC terminal 210_3 are illustrated as examples of user terminals, but the present disclosure is not limited thereto, and the user terminals 210_1, 210_2, and 210_3 may be any computing devices capable of wired and/or wireless communication and on which the brain image quantitative analysis service application or the web browser can be installed and executed. For example, the user terminal may include an AI speaker, a smartphone, a mobile phone, a navigation device, a computer, a laptop, a digital broadcasting terminal, Personal Digital Assistants (PDA), a Portable Multimedia Player (PMP), a tablet PC, a game console, a wearable device, an IoT (internet of things) device, a VR (virtual reality) device, an AR (augmented reality) device, a set-top box, and the like. Also, although FIG. 2 illustrates that three user terminals 210_1, 210_2, and 210_3 communicate with the information processing system 230 via the network 220, the present disclosure is not limited thereto, and a different number of user terminals may be configured to communicate with the information processing system 230 via the network 220.

An external device 240 may communicate with the information processing system 230 via the network 220. Here, the external device 240 may be a brain image capturing device for providing the brain image quantitative analysis service. The external device 240 may transmit a brain image (e.g., a CT image, a PET image, an MRI image) to the information processing system 230 via the network 220. Although FIG. 2 illustrates that the information processing system 230 receives the brain image from the external device 240 via the network 220, the present disclosure is not limited thereto. For example, the external device 240 and the information processing system 230 may be configured as a single device or system.

Although FIG. 2 exemplarily illustrates a configuration in which the user terminals 210_1, 210_2, and 210_3 are provided with the brain image quantitative analysis service by communicating with the information processing system 230, the present disclosure is not limited thereto, and a request or input of the user may be provided to the information processing system 230 through an input device associated with the information processing system 230 without going through the user terminal 210_1, 210_2, or 210_3, and a result of processing the request or input of the user may be provided to the user through an output device (e.g., a display, etc.) associated with the information processing system 230.

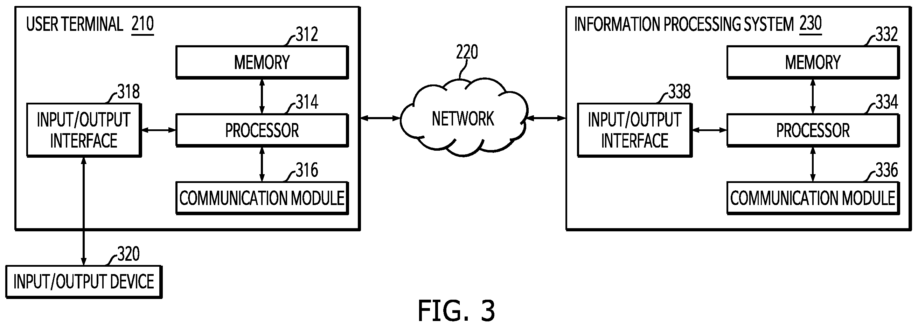

FIG. 3 is a block diagram illustrating internal configurations of a user terminal 210 and the information processing system 230 according to an embodiment of the present disclosure. The user terminal 210 may refer to any computing device capable of executing an application, a web browser, and the like and capable of wired/wireless communication, and may include, for example, the mobile phone terminal 210_1, the tablet terminal 210_2, the PC terminal 210_3, and the like in FIG. 2. As illustrated, the user terminal 210 may include a memory 312, a processor 314, a communication module 316, and an input/output interface 318. Similarly, the information processing system 230 may include a memory 332, a processor 334, a communication module 336, and an input/output interface 338. As illustrated in FIG. 3, the user terminal 210 and the information processing system 230 may be configured to communicate information and/or data via the network 220 using the respective communication modules 316 and 336. Also, an input/output device 320 may be configured to input information and/or data to the user terminal 210 through the input/output interface 318 or to output information and/or data generated from the user terminal 210.

The memories 312 and 332 may include any non-transitory computer-readable recording medium. According to an embodiment, the memories 312 and 332 may include a permanent mass storage device such as a read only memory (ROM), a disk drive, a solid state drive (SSD), and flash memory. As another example, a non-volatile mass storage device such as a ROM, an SSD, flash memory, and a disk drive may be included in the user terminal 210 or the information processing system 230 as a separate permanent storage device distinct from the memory. Also, an operating system and at least one program code may be stored in the memories 312 and 332.

Such software components may be loaded from a computer-readable recording medium separate from the memories 312 and 332. Such a separate computer-readable recording medium may include a recording medium directly connectable to the user terminal 210 and the information processing system 230, which may include, for example, a computer-readable recording medium such as a floppy drive, a disk, a tape, a DVD/CD-ROM drive, and a memory card. As another example, the software components may be loaded into the memories 312 and 332 through the communication modules 316 and 336 rather than the computer-readable recording medium. For example, at least one program may be loaded into the memories 312 and 332 based on a computer program installed by files provided via the network 220 by developers or a file distribution system distributing installation files of an application.

The processors 314 and 334 may be configured to process instructions of a computer program by performing basic arithmetic, logic, and input/output operations. The instructions may be provided to the processors 314 and 334 by the memories 312 and 332 or the communication modules 316 and 336. For example, the processors 314 and 334 may be configured to execute received instructions according to program code stored in a recording device such as the memories 312 and 332.

The communication modules 316 and 336 may provide a configuration or function for the user terminal 210 and the information processing system 230 to communicate with each other via the network 220, and may provide a configuration or function for the user terminal 210 and/or the information processing system 230 to communicate with another user terminal or another system (for example, a separate cloud system, etc.). For example, a request or data (e.g., a brain image quantitative analysis request, etc.) generated by the processor 314 of the user terminal 210 according to program code stored in a recording device such as the memory 312 may be delivered to the information processing system 230 via the network 220 under control of the communication module 316. Conversely, a control signal or command provided under control of the processor 334 of the information processing system 230 may be received by the user terminal 210 through the communication module 316 of the user terminal 210 via the communication module 336 and the network 220.

The input/output interface 318 may be a means for interfacing with the input/output device 320. As an example, an input device may include devices such as a camera including an audio sensor and/or an image sensor, a keyboard, a microphone, and a mouse, and an output device may include devices such as a display, a speaker, and a haptic feedback device. As another example, the input/output interface 318 may be a means for interfacing with a device in which configurations or functions for performing input and output are integrated into one, such as a touch screen. For example, as the processor 314 of the user terminal 210 processes instructions of a computer program loaded into the memory 312, a service screen configured using information and/or data provided by the information processing system 230 or another user terminal may be displayed on a display through the input/output interface 318. Although FIG. 3 illustrates that the input/output device 320 is not included in the user terminal 210, the present disclosure is not limited thereto, and the input/output device 320 may be configured as one device with the user terminal 210. Also, the input/output interface 338 of the information processing system 230 may be a means for interfacing with a device (not shown) for input or output that is connected to the information processing system 230 or that the information processing system 230 may include. Although FIG. 3 illustrates the input/output interfaces 318 and 338 as elements configured separately from the processors 314 and 334, the present disclosure is not limited thereto, and the input/output interfaces 318 and 338 may be configured to be included in the processors 314 and 334.

The user terminal 210 and the information processing system 230 may include more components than the components in FIG. 3. However, there is no need to clearly show most conventional components. In an embodiment, the user terminal 210 may be implemented to include at least some of the input/output devices 320 described above. Also, the user terminal 210 may further include other components such as a transceiver, a Global Positioning System (GPS) module, a camera, various sensors, and a database.

While a program for a brain image quantitative analysis service application or the like is operating, the processor 314 may receive text, an image, a video, voice, and/or an action input or selected through an input device such as a touch screen, a keyboard, a camera including an audio sensor and/or an image sensor, or a microphone connected to the input/output interface 318, and may store the received text, the image, the video, the voice, and/or the action in the memory 312 or provide the received text, the image, the video, the voice, and/or the action to the information processing system 230 through the communication module 316 and the network 220.

The processor 314 of the user terminal 210 may be configured to manage, process, and/or store information and/or data received from the input/output device 320, another user terminal, the information processing system 230, and/or a plurality of external systems. Information and/or data processed by the processor 314 may be provided to the information processing system 230 through the communication module 316 and the network 220. The processor 314 of the user terminal 210 may transmit information and/or data to the input/output device 320 through the input/output interface 318 to output the information and/or data. For example, the processor 314 may output or display the received information and/or data on a screen of the user terminal 210.

The processor 334 of the information processing system 230 may be configured to manage, process, and/or store information and/or data received from the plurality of user terminals 210 and/or a plurality of external systems. Information and/or data processed by the processor 334 may be provided to the user terminal 210 through the communication module 336 and the network 220.

FIG. 4 illustrates an example of a method for training a CT parcellation model 450 according to an embodiment of the present disclosure. As described above in FIG. 1, the CT parcellation model 450 may be a model trained using, as a training data set, labeling information 420 extracted by parcellating a brain MRI image 410 and a brain CT image 430.

According to an embodiment, a processor may receive the brain MRI image 410 and the brain CT image 430 of a same patient from an external device. Here, the external device may be a brain image capturing device. For example, the external device may include an MRI device, a CT device, and a PET/CT device, but is not limited thereto. Also, an MRI image received from the external device may include a T1-weighted MRI image, a T2-weighted MRI image, a Fluid-attenuated inversion recovery (FLAIR) MRI image, and the like, but is not limited thereto.

According to an embodiment, the processor may perform a parcellation task (parcellation) 412 on the brain MRI image 410. In this case, the processor may perform the parcellation task on an entire brain tissue included in the brain MRI image 410. That is, the processor may perform the parcellation task on the entire brain tissue rather than segmenting only a specific part of a brain (for example, a brain tumor). Thereafter, the processor may extract each of a plurality of regions for the brain tissue included in the brain MRI image 410.

According to an embodiment, MRI image-based labeling information 420 may be generated for the extracted plurality of regions. In this case, the labeling information 420 may include parcellation information for at least one anatomical region of interest for which a brain quantitative analysis result is to be obtained. The MRI image-based labeling information 420 may be generated by an auto labeling method using an MRI Segmentation Model, but the present disclosure is not limited thereto. Here, a segmentation model performing the auto labeling may use a deep learning-based algorithm, volume-based morphometry, surface-based morphometry, or the like, but is not limited thereto. Also, the MRI image-based labeling information 420 may be generated by a method in which an actual person performs manual labeling on the MRI image.

According to an embodiment, the processor may spatially register (co-registration) 440 the brain MRI image 410 and the brain CT image 430 into a same coordinate system. In this case, because the brain MRI image 410 and the brain CT image 430 are brain images of the same patient, the brain MRI image 410 and the brain CT image 430 may be registered 440 by a rigid-body transformation method. For example, the brain MRI image 410 and the brain CT image 430 may be registered 440 through rotation and/or translation. In this case, the processor may use a multi-modal coregistration algorithm as described above in FIG. 1, but is not limited thereto.

According to an embodiment, the processor may set a weight for each of the plurality of regions included in the brain MRI image 410 based on the labeling information 420 acquired from the brain MRI image 410. Here, the weight may be determined to be inversely proportional to an area of each of the plurality of regions included in the brain MRI image 410. For example, among the plurality of regions extracted from the brain MRI image 410, a weight of a region with a large area may be determined to be low, and a weight of a region with a small area may be determined to be high.

According to an embodiment, the weight for each of the plurality of regions included in the brain MRI image 410 may be determined to be greater than or equal to a predetermined threshold. That is, among the plurality of regions included in the brain MRI image 410, a minimum value of the weight may be determined to be greater than or equal to the predetermined threshold even for a region with a largest area. Also, a weight greater than or equal to the predetermined threshold may be determined not only for the plurality of regions included in the brain MRI image 410 but also for a background area.

According to an embodiment, the processor may construct a training data set with the labeling information 420 acquired from the brain MRI image 410 and the brain CT image 430 as a pair. In this case, the CT parcellation model 450 may be trained by applying the weight determined to be greater than or equal to the predetermined threshold to each of the plurality of regions included in the brain MRI image 410. According to this configuration, the CT parcellation model 450 may be uniformly trained for all regions regardless of the size of the area of the region extracted from the brain MRI image 410.

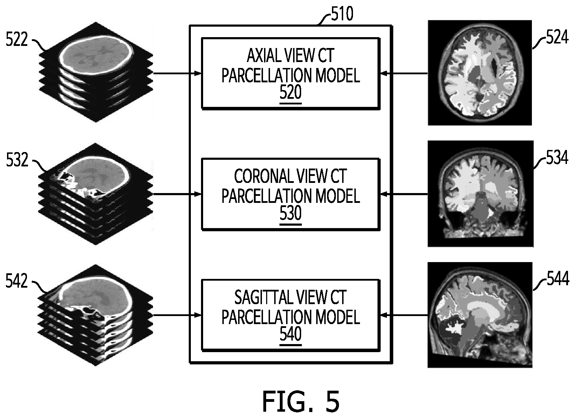

FIG. 5 illustrates an example of a method for training a CT parcellation model 510 using two-dimensional brain images according to an embodiment of the present disclosure. As described above in FIG. 4, the CT parcellation model 510 may be a model trained using, as a training data set, labeling information (524, 534, and 544) extracted by parcellating brain MRI images and brain CT images (522, 532, and 542).

According to an embodiment, the CT parcellation model 510 may use a two-dimensional or three-dimensional U-net structure network based on a Convolutional Neural Network (CNN), a transformer, or the like, but is not limited thereto. Specifically, a deep learning-based model may perform training and/or inference by dividing a three-dimensional brain MRI image and a brain CT image into two-dimensional slices. In this case, three deep learning-based models may be trained based on slices cut from the three-dimensional MRI image into an axial view, a coronal view, and a sagittal view, respectively.

Specifically, a processor may receive a three-dimensional brain CT image from an external device. Thereafter, the processor may extract a two-dimensional axial view CT image 522, a two-dimensional coronal view CT image 532, and a two-dimensional sagittal view CT image 542 from the three-dimensional brain CT image, respectively. In FIG. 5, the axial view CT image 522, the coronal view CT image 532, and the sagittal view CT image 542 are illustrated as five each, but the present disclosure is not limited thereto. For example, each of the axial view CT image 522, the coronal view CT image 532, and the sagittal view CT image 542 may be greater than or equal to one and less than or equal to four. Also, the axial view CT image 522, the coronal view CT image 532, and the sagittal view CT image 542 may be greater than or equal to six.

Specifically, it can be assumed that the three-dimensional brain CT image includes 100 axial 2D slices, and training and/or inference for a 7th slice among the 100 slices is performed. In this case, referring to FIG. 5, a slice adjacent to the 7th slice may represent a middle slice among the five axial view CT images 522 in FIG. 5. Accordingly, remaining four slices of the five axial view CT images 522 may represent a 5th slice, a 6th slice, an 8th slice, and a 9th slice. That is, the five axial view CT images 522 may include five adjacent slice images with the 7th slice as a middle image. Although this example illustrates putting five slices as images, how many adjacent slices to use as a CT image input may be arbitrarily set. Also, such an operation may be applied not only to an axial view CT parcellation model 520 but also to a coronal view CT parcellation model 530 and a sagittal view CT parcellation model 540. Under this configuration, each model may perform inference for a middle slice based on contextual information by referring to adjacent slices.

Also, the processor may receive a three-dimensional brain MRI image from the external device. For the received MRI image, an MRI image corresponding to a CT image to be analyzed among a plurality of CT images may be input. For example, in FIG. 5, a middle CT image among five images included in each of the axial view CT image 522, the coronal view CT image 532, and the sagittal view CT image 542 may be an analysis target image, and the input MRI image may be an image corresponding to the middle CT image.

Thereafter, the processor may perform a parcellation task on the brain MRI image to extract each of a plurality of regions included in the brain MRI image. Thereafter, the processor may generate three-dimensional labeling information for the extracted plurality of regions. Additionally, the processor may extract two-dimensional axial view labeling information 524, two-dimensional coronal view labeling information 534, and two-dimensional sagittal view labeling information 544 from the three-dimensional labeling information.

According to an embodiment, the processor may construct a training data set with the axial view CT image 522 extracted from the brain CT image and the axial view labeling information 524 extracted from the brain MRI image as a pair. Similarly, the processor may construct a training data set with the coronal view CT image 532 extracted from the brain CT image and the coronal view labeling information 534 extracted from the brain MRI image as a pair, and a training data set with the sagittal view CT image 542 extracted from the brain CT image and the sagittal view labeling information 544 extracted from the brain MRI image as a pair, respectively.

According to an embodiment, the CT parcellation model 510 may include the axial view CT parcellation model 520, the coronal view CT parcellation model 530, and the sagittal view CT parcellation model 540. Accordingly, the axial view CT parcellation model 520 may be trained using the training data set of the axial view CT image 522 and the axial view labeling information 524. Similarly, the coronal view CT parcellation model 530 may be trained using the training data set of the coronal view CT image 532 and the coronal view labeling information 534, and the sagittal view CT parcellation model 540 may be trained using the training data set of the sagittal view CT image 542 and the sagittal view labeling information 544, respectively.

According to an embodiment, the processor may be trained to generate labeling information based on the three-dimensional brain CT image received using the CT parcellation model 510. Specifically, the axial view CT parcellation model 520 may be trained to generate axial view labeling information using a brain axial view CT image extracted from the received brain CT image as an input value. Similarly, the coronal view CT parcellation model 530 may be trained to generate coronal view labeling information using a brain coronal view CT image extracted from the received brain CT image as an input value, and the sagittal view CT parcellation model 540 may be trained to generate sagittal view labeling information using a brain sagittal view CT image extracted from the received brain CT image as an input value.

According to an embodiment, the processor may generate labeling information for a parcellated region within one MRI image for a plurality of given inference target CT images (i.e., a plurality of axial view CT images, a plurality of coronal view CT images, and a plurality of sagittal view CT images) using the three CT parcellation models trained as described above. Three-dimensional labeling information may be generated by synthesizing the axial view labeling information, the coronal view labeling information, and the sagittal view labeling information generated in this manner. Specifically, the processor may generate one three-dimensional labeling information by completing a three-dimensional probability map by ensembling the axial view labeling information, the coronal view labeling information, and the sagittal view labeling information.

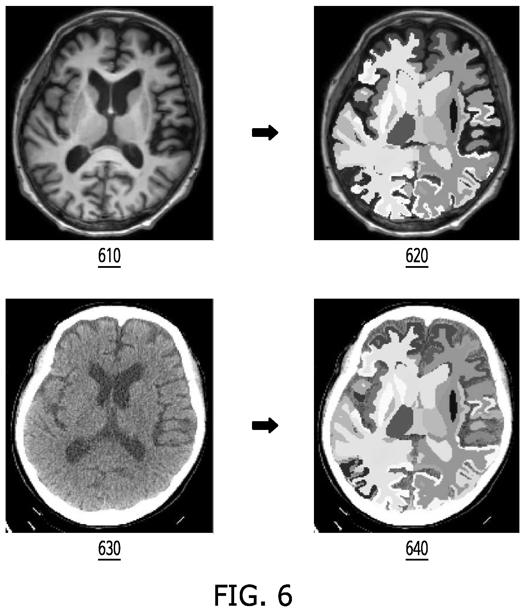

FIG. 6 illustrates an example of labeling information 620 generated based on an MRI image 610 and labeling information 640 generated based on a CT image 630 according to an embodiment of the present disclosure. According to an embodiment, a processor may perform a parcellation task on a brain image. Thereafter, the processor may extract each of a plurality of regions for brain tissue included in the brain image to generate labeling information. In this case, the labeling information may include parcellation information for at least one region of interest for which a brain quantitative analysis result is to be obtained.

Labeling information 620 is an example of labeling information generated based on the brain MRI image 610. The processor may train a CT parcellation model using the labeling information 620 generated based on the brain MRI image 610. Specifically, the processor may perform a parcellation task on the received MRI image 610 to generate the labeling information 620 based on the brain MRI image. Thereafter, the generated labeling information 620 may be used as training data for the CT parcellation model together with a brain CT image of a patient who captured the brain MRI image 610.

Labeling information 640 is an example of labeling information generated based on the brain CT image 630. The processor may receive the brain CT image 630 and generate the labeling information 640. Specifically, the processor may input the received brain CT image 630 into the CT parcellation model. Thereafter, the CT parcellation model may generate and output CT image-based labeling information 640 using the brain CT image 630 as an input value.

Referring to FIG. 6, the brain CT image 630 may have lower resolution and contrast compared to the brain MRI image 610. Accordingly, in a case where labeling information is generated by directly performing a parcellation task on the brain CT image 630, it may be difficult to generate detailed labeling information at a level generated from the brain MRI image 610. Also, a possibility of error occurrence in the parcellation task may increase. However, in a case where the labeling information 640 for the brain CT image 630 is generated using the CT parcellation model trained based on the brain MRI image 610, detailed labeling information at a level generated from the brain MRI image 610 may be generated. That is, biological information having high resolution and contrast at a level obtainable from an MRI image may be acquired with only the CT image.

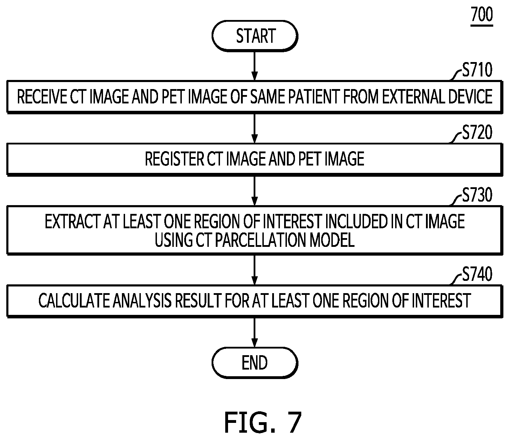

FIG. 7 is a flowchart illustrating an example of a method 700 for quantitative analysis of brain images according to an embodiment of the present disclosure. In an embodiment, the method 700 may be performed by at least one processor (e.g., a processor of an information processing system, etc.). The method 700 may be initiated by the processor receiving a CT image and a PET image of a same patient from an external device (S710). Thereafter, the processor may register the CT image and the PET image (S720). The processor may extract at least one region of interest included in the CT image using a CT parcellation model (S730). In this case, the CT parcellation model may have been trained using a predetermined training data set. Here, the predetermined training data set may have been generated based on a CT image and an MRI image of the same patient.

According to an embodiment, when training the CT parcellation model using the predetermined training data set, the method may include receiving the CT image and the MRI image of the same patient, generating labeling information by performing a parcellation task (parcellation) on the MRI image to extract each of a plurality of regions included in the MRI image, and constructing a training data set with the labeling information and the CT image as a pair. Here, the constructing of the training data set may include spatially registering the MRI image and the CT image.

According to an embodiment, the training of the CT parcellation model may include applying a weight to each of a plurality of regions included in the MRI image. In this case, the weight may be determined to be inversely proportional to an area of each of the plurality of regions included in the MRI image. Also, the weight may be determined to be greater than or equal to a predetermined threshold.

According to an embodiment, the CT parcellation model may include an axial view CT parcellation model, a coronal view CT parcellation model, and a sagittal view CT parcellation model. Also, the axial view CT parcellation model may be trained based on an axial view CT image and labeling information of an axial view MRI image, the coronal view CT parcellation model may be trained based on a coronal view CT image and labeling information of a coronal view MRI image, and the sagittal view CT parcellation model may be trained based on a sagittal view CT image and labeling information of a sagittal view MRI image. Accordingly, the extracting of the at least one region of interest may include generating three-dimensional labeling information of the CT image by synthesizing respective labeling information generated by the axial view CT parcellation model, the coronal view CT parcellation model, and the sagittal view CT parcellation model.

According to an embodiment, the processor may calculate an analysis result (for example, an analysis result for the PET image, etc.) for the extracted at least one region of interest using the CT parcellation model (S740). In this case, the calculating of the analysis result may include calculating a standardized uptake value ratio (SUVR) of the at least one region of interest.

The above-described method may be provided as a computer program stored in a computer-readable recording medium for execution in a computer. The medium may continuously store a computer-executable program, or temporarily store the program for execution or download. Also, the medium may be various recording means or storage means in a form in which a single hardware component or several hardware components are combined, and the medium is not limited to a medium directly connected to a certain computer system, but may be distributed on a network. Examples of the medium may include magnetic media such as a hard disk, a floppy disk, and magnetic tape, optical recording media such as a CD-ROM and a DVD, magneto-optical media such as a floptical disk, and media configured to store program instructions, including a ROM, a RAM, flash memory, and the like. Also, as examples of other media, there may be recording media or storage media managed by an app store distributing applications, sites supplying or distributing various other software, servers, and the like.

The methods, operations, or techniques of the present disclosure may be implemented by various means. For example, these techniques may be implemented in hardware, firmware, software, or a combination thereof. Those of ordinary skill in the art will understand that various illustrative logical blocks, modules, circuits, and algorithm steps described in connection with the disclosure herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design requirements imposed on the overall system. Those of ordinary skill in the art may implement the described functionality in varying ways for each particular application, but such implementations should not be interpreted as causing a departure from the scope of the present disclosure.

In a hardware implementation, processing units used to perform the techniques may be implemented within one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, a computer, or a combination thereof.

Accordingly, various illustrative logic blocks, modules, and circuits described in connection with the present disclosure may be implemented or performed with a general-purpose processor, a DSP, an ASIC, an FPGA or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. The processor may also be implemented as a combination of computing devices, for example, a combination of a DSP and a microprocessor, a plurality of microprocessors, one or more microprocessors in conjunction with a DSP core, or any other such configuration.

In firmware and/or software implementations, the techniques may be implemented as instructions stored on a computer-readable medium such as random access memory (RAM), read-only memory (ROM), non-volatile random access memory (NVRAM), programmable read-only memory (PROM), erasable programmable read-only memory (EPROM), electrically erasable PROM (EEPROM), flash memory, a compact disc (CD), a magnetic or optical data storage device, and the like. The instructions may be executable by one or more processors and may cause the processor(s) to perform certain aspects of the functionality described in the present disclosure.

When implemented in software, the techniques may be stored on or transmitted through a computer-readable medium as one or more instructions or code. Computer-readable media include both computer storage media and communication media including any medium that facilitates transfer of a computer program from one place to another. Storage media may be any available media that can be accessed by a computer. By way of non-limiting example, such computer-readable media may include RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium.

For example, if software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, include CD, laser disc, optical disc, digital versatile disc (DVD), floppy disk, and Blu-ray disc, where disks usually reproduce data magnetically, while discs reproduce data optically with lasers. Combinations of the above should also be included within the scope of computer-readable media.

A software module may reside in RAM memory, flash memory, ROM memory, EPROM memory, EEPROM memory, registers, a hard disk, a removable disk, a CD-ROM, or any other form of storage medium known in the art. An exemplary storage medium may be coupled to the processor such that the processor can read information from and write information to the storage medium. In the alternative, the storage medium may be integrated into the processor. The processor and the storage medium may reside in an ASIC. The ASIC may reside in a user terminal. In the alternative, the processor and the storage medium may reside as discrete components in the user terminal.

Although the embodiments described above have been described as utilizing aspects of the presently disclosed subject matter in one or more standalone computer systems, the present disclosure is not limited thereto, and may be implemented in connection with any computing environment such as a network or distributed computing environment. Furthermore, aspects of the subject matter in the present disclosure may be implemented in a plurality of processing chips or devices, and storage may be similarly affected across a plurality of devices. Such devices may include PCs, network servers, and portable devices.

Although the present disclosure has been described in connection with some embodiments in the present specification, various modifications and changes can be made without departing from the scope of the present disclosure that can be understood by those of ordinary skill in the art to which the invention of the present disclosure belongs. Also, such modifications and changes should be considered to fall within the scope of the claims attached to the present specification.

## Claims

1. A method for quantitative analysis of brain images based on computed tomography (CT) images, performed by at least one processor, the method comprising:
receiving a CT image and a positron emission tomography (PET) image of a same patient from an external device;
registering the CT image and the PET image;
extracting at least one region of interest included in the CT image using a CT parcellation model; and
calculating an analysis result for the at least one region of interest.

2. The method as claimed in claim 1, wherein the calculating of the analysis result comprises calculating a standardized uptake value ratio (SUVR) of the at least one region of interest.

3. The method as claimed in claim 1, further comprising training the CT parcellation model using a predetermined training data set, wherein the predetermined training data set is generated based on a CT image and an MRI image of the same patient.

4. The method as claimed in claim 3, wherein the training of the CT parcellation model using the predetermined training data set comprises:
receiving the CT image and the MRI image of the same patient;
generating labeling information of a plurality of regions included in the MRI image by performing a parcellation task on the MRI image;
constructing a training data set with the labeling information and the CT image as a pair; and
training the CT parcellation model using the training data set.

5. The method as claimed in claim 4, wherein the constructing the training data set comprises spatially registering the MRI image and the CT image.

6. The method as claimed in claim 4, wherein the training the CT parcellation model comprises applying a weight to each of a plurality of regions included in the MRI image, and
the weight is determined to be inversely proportional to an area of each of the plurality of regions included in the MRI image.

7. The method as claimed in claim 6, wherein the weight is determined to be greater than or equal to a predetermined threshold.

8. The method as claimed in claim 4, wherein the CT parcellation model comprises an axial view CT parcellation model, a coronal view CT parcellation model, and a sagittal view CT parcellation model, and
the axial view CT parcellation model is trained based on an axial view CT image and labeling information of an axial view MRI image,
the coronal view CT parcellation model is trained based on a coronal view CT image and labeling information of a coronal view MRI image, and
the sagittal view CT parcellation model is trained based on a sagittal view CT image and labeling information of a sagittal view MRI image.

9. The method as claimed in claim 8, wherein the extracting the at least one region of interest comprises generating three-dimensional labeling information of the CT image by synthesizing respective labeling information generated by the axial view CT parcellation model, the coronal view CT parcellation model, and the sagittal view CT parcellation model.

10. A computer-readable non-transitory recording medium recording instructions for executing the method according to any one of claims 1 to 9 in a computer.

11. A system comprising:
a communication module;
a memory; and
at least one processor connected to the memory and configured to execute at least one computer-readable program included in the memory,
wherein the at least one program comprises instructions for:
receiving a CT image and a PET image from an external device;
registering the CT image and the PET image;
extracting at least one region of interest included in the CT image using a CT parcellation model; and
calculating an analysis result for the at least one region of interest.
